# EUROPEAN PATENT SPECIFICATION

(11) **EP 1 746 966 B1**
(45) Date of publication and mention of the grant of the patent: **23.04.2014**
(21) Application number: 05707209.2
(22) Date of filing: 04.02.2005
(51) Int. Cl.: A61K 6/06, A61K 6/083, A61K 6/087, A61L 27/12, A61L 27/46, A61K 33/16, A61K 33/42

(54) **COMPOSITION CONTAINING NANO-CRYSTALLINE APATITE**
ZUSAMMENSETZUNG MIT NANOKRISTALLINEM APATIT
COMPOSITION CONTENANT DE L'APATITE NANO-CRISTALLINE

(30) Priority: 18.05.2004 DE 102004025030
(43) Date of publication of application: 31.01.2007
(73) Proprietor: S & C Polymer Silicon- und Composite-Spezialitäten GmbH, 25335 Elmshorn (DE); Merz Dental GmbH, 24321 Lütjenburg (DE)
(72) Inventor: ENGELBRECHT, Jürgen, 22607 Hamburg (DE); DZIURON, Peter, 24327 Biekendorf (DE)
(74) Representative: Boeters, Hans Dietrich
(86) International application number: PCT/EP2005/001159
(87) International publication number: WO 2005/110339

(56) References cited:
- WO-A-00/03747
- WO-A-01/01930
- WO-A-01/95863
- US-A- 5 952 399
- WILLIGEROTH, S.F., BENEKE K., HANNIG, M., ZIMEHL, R.: "Preparation strategies for phosphate-based mineral biomaterials" PROGRESS IN COLLOID & POLYMER SCIENCE, vol. 121, 2002, pages 1-6, XP008045516

## Description

### Introduction

This invention describes compositions containing nano-crystalline apatite that are useful as bone- or preferably as tooth restorative materials. The materials produced, using the composition have improved properties in the areas of esthetics, hardness, translucency, surface polishability, strength and the capability to release and to take ions up in respect of a biological environment.

### State of the Art

Due to their chemical and structural similarity to biological hard tissue synthetic orthophosphates, especially apatites like Ca₁₀(PO₄)₆(OH)₂ or Ca₁₀(PO₄)₆F₂, are of special interest as materials for medical applications, alone as well as in combination with other materials like polymers, gasses etc.

US 4,778,834 describes dental materials that are prepared by combining hydroxyapatite fillers, Ca₁₀(PO₄)₆(OH)₂, with particle sizes in the µm-range, with polymerizable monomers in concentrations up to 70%. In contrast to silica- or silicate-like fillers, apatite fillers cannot be silanized for the purpose of an increased mechanical bonding with the polymerizable resin matrix. However, before mixing, the hydroxyapatite particles can be coated with a silicate cover and then silanized with a standard method to prepare dental materials. A disadvantage of such prepared materials is the restricted ion exchange (calcium, phosphate, etc.) with the environment due to the silicate cover.

Another drawback is the difficulty in achieving the transparency required for dental filling materials. This is because the refractive index of 1.62 for hydroxyapatite Ca₁₀(PO₄)₆(OH)₂ is too high compared to values between 1.45 and 1.53 for the polymer components of the resin matrix. This difference in the refractive indices results in unesthetic, opaque materials with low translucence so that the usual photopolymerization of these materials is only possible in thin layers. Furthermore the materials are not capable of releasing fluoride ions to prevent caries - a desirable property of dental materials.

WO A 94/23944 describes dental materials that are made by combining polymerizable monomers with crystalline, sintered fluoroapatite Ca₁₀(PO₄)₆ F₂ with particle sizes in the sub-micron-to micron range, in particular 0.5 - 0.7 µm, in concentrations between 10 and 70 % by volume. These dental materials release fluoride ions into their biological environment but they do not have the required translucency acceptable for dental materials because, as already mentioned, the refractive index of 1.63 for fluorapatite Ca₁₀(PO₄)₆ F₂ is too high compared to the values for the polymeric resin matrix. This results in opaque materials with poor esthetics and low photopolymerization depth of cure as a result of low light penetration.

To overcome this deficiency Antonucci, J. M. Dent. Mat. 7:124 - 129, 1991 proposed the use of calcium-metaphosphates (Ca(PO₃)₂)ₙ instead of fluorapatite to attain a decrease of the refractive index to 1.54 - 1.59. These fillers, in combination with selected resin matrixes, yield dental materials with an improved transparency. However, they show higher solubility in comparison to fluorapatite and due to this a lower stability in the oral environment. In addition, they do not release fluoride ions.

Therefore, with respect to the work by Antonucci, it can be concluded that translucent dental materials prepared by the addition of fluoride-donors like fluorapatite, having low solubility but refractive indices in the same range or lower as with calcium-metaphosphates are especially desirable.

EP 0 193 588 B1 describes carbonate-substituted apatites with grain sizes bigger than 0.1 mm for the repair of bone and teeth defects. These carbonate-substituted apatites lack tooth-like translucency and the required strength to be suitable as filling material in high occlusal load bearing applications. Due to their high opacities these materials are not light-curable. Instead, self-curable compositions, acceptable only for implantation purposes, were obtained.

DE 23 26 100 B2 and DE 25 01 683 A1 describe bioactive adhesive materials used for prosthetic purposes, in particular two-phase glasses made of a crystalline apatite-phase embedded in a glass phase. Such materials can be silanized, but when used with common acrylic resins, this results in an opacification of the obtained material. Also described are low carbonate-substituted apatites, characterized by poor optical properties. Due to the lack of radiopacity of the described two-phase glasses, the resulting acrylated-apatite/glass material, not surprisingly, is unsuitable as a dental restorative material.

US 5,304,577 is solely concerned with phophate-apatites with particle sizes in the range of 2 - 10 µm. Strontium phosphate-apatite, just as calcium phosphate-apatite, has a refractive index of 1.63. Therefore, the desired decrease in refractive index could not be achieved through the substitution of calcium with strontium. Therefore, the authors came to the conclusion that these materials were only suitable for less visible areas, i. e. in bone defects, in root canals, in periodontics. In summary, the described dental materials were characterized by unacceptably high opacity combined with insufficient strength to make them suitable for the repair of the more visible high load-bearing areas.

Derwent-Abstract no. 85-021 799 [04] and Derwent-Abstract no. 85-022 140 [04] describe the preparation of opaque pastes resulting from the reaction of pure phosphate-apatites with dimethacrylates. Due to low light penetration, these materials are not readily photopolymerizable. This also suggests that they are of unacceptably high opacity. Therefore, two paste systems, e. g. amine/peroxide, have to be formulated for satisfactory curing. Since apatite was the only filler, the strength of the resulting materials was too low. In addition to this deficiency, the described cation-substitutions employed in the patent also did not lead to a decrease of the refractive index.

EP 0 832 636 A2 describes the application of fluoride-containing mixed apatites in polymerisable dental materials, wherein these mixed apatites comprise combinations of similarly or equally charged cations, or identical or different sulfate, fluorophosphate or carbonate anions as well as fluoride, chloride, hydroxide or oxide anions, with refractive indices and particle sizes in the range of 1.52-1.66 (refr.ind.) and 0.01 µm and 10 µm (part.size) respectively. By these additions of mixed apatites the translucency of the materials was improved. However, the effect of increased solubility of the mixed apatite (in comparison to the pure apatite) on the stability of the polymerized material in the oral environment was not discussed.

In WO 9530402, Wictorin has described, amongst other fillers, the use of micron-sized apatite as filler in curable epoxy-formulated dental resin composite filling materials. Due to the similarity in refractive indices of the epoxy- and the methacrylate-resins, the resulting dental resin composite prepared from the epoxy-resins were of similarly unacceptable high opacity. Also, due to the poor adhesion between the filler and the resin matrix, the mechanical properties of the resulting composite were insufficient.

WO 97/17285 describes mixtures of amorphous and nano crystalline calcium phosphate apatite in different physical forms in particle sizes > 250 µm used as resorbable synthetic bone material.

In D. Res. Nat. Stand. Technol., 108, 167-182 (2003), D. Skritic and D. M. Antonucci describe the use of amorphous calcium phosphate, which is known as the precursor of biological apatite, as filling material in the preparation of bioactive polymerisable resin composites. Although the bioactivity of theses materials is desirable, due to the amorphous µm - mm sized agglomerated filler structure, the stability, the esthetics and the mechanical properties of the resulting resin composites are poor.

WO 00/03747 describes non-hardenable dental compositions containing nano-crystalline apatite sized from 0,0005 to 0,2 µm. The main purpose of these apatite compositions is to stimulate the remineralization of enamel and dentin. In addition, due to their extreme small size, these nano particles are able to penetrate deep into the dentinal tubules.

In Progr. Colloid Polym. Sci., 121, 1-6 (2002), S.F. Willigeroth, K. Beneke, M. Hannig and R. Zimehl describe the preparation of nanocrystalline fluoroapatite fillers.

Thus, in the state of the art there is no solution of the problem to get hardenable materials suitable for applications as bone- and especially as tooth restorative materials which have improved properties in the areas of esthetics, hardness, translucency, surface polishability, strength and the capability to release ions and to take them up in a biological environment. Either the known products are too opaque in the cured state or their mechanical properties are insufficient or the ion transfer between restorative matrial and biological environment is hindered.

Therefore it was the object of the present invention to provide improved materials that are useful as bone- and especially as tooth restorative materials, overcoming disadvantages of the prior art.

This object is solved by the provision of a hardenable composition, which can be used as bone- and especially as tooth restorative material, having improved properties in the areas of esthetics, hardness, translucency, surface polishability, strength and the capability to release ions and to take them up in a biological environment. The inventive composition comprises a mixture of a crosslinkable resin and/or hardenable acid/base cement system that contains nanocrystalline apatite of the following general formula:

Ca₁₀(PO₄)₆A_{z}(OH)_{2-z}

wherein A is F⁻ and wherein at least 50 wt-% of the apatite-particles have a particle size within the range of from 2 to 200 nm, and wherein z=0 (Hydroxyapatite or z=2 (Fluoroapatite).

### Description of the invention

The composition, optionally in combination with additional components, provides for materials having the desired tooth-like transparency, good mechanical properties, the desired ion-release and ion-uptake properties. Preferred additional components are other fillers, e.g. glasses or microfine silicic acid, commonly used dental monomers and/or dental cement systems. The improved refractive index of the inventive material is probably due to the size of the apatite particles. With more than 50% by weight (wt.-%) of the particles having a particle size smaller than 200 nm, they are incapable of scattering visible light of a wavelength between 400 nm and 900 nm. This feature is a major advantage of the inventive material.

The materials provided by the present invention can be used as bone cements or bone substitutes, and especially as fillings, inlays or onlays, luting cements, veneers for crowns and bridges, materials for artificial tooth, dentine- and enamel bonding agents, underfilling materials, core build-up materials, sealants, root canal filling materials or other miscellaneous curable materials in prosthetic, conservative and preventive dentistry.

The inventive composition contains hardenable matrices (crosslinkable resins and/or acid/base-cements) and nano apatites and optionally other filler materials and miscellaneous additives.

The inventive composition can be based on a curable matrix which is prepared from polymerizable ethylene-based unsaturated monomers, preferably containing acrylic- and/or methacrylic groups.

As polymerizable monomers, all those polymerizable monomers based on ethylenically unsaturated monomers known to a person skilled in the art can be used for this application. The polymerizable monomers preferably include those having acrylic and/or methacrylic groups.

Preferred polymerizable monomers are: esters of α-cyanoacrylic acid,(meth)acrylic acid, urethane (meth) acrylic acid, crotonic acid, cinnamic acid, sorbic acid, maleic acid and itaconic acid with monohydric or dihydric alcohols; (meth) acrylamides such as, for example, N-isobutyl acrylamide; vinyl esters of carboxylic acids such as, for example, butyl vinyl ether; mono-N-vinyl compounds such as N-vinyl pyrrolidone; and styrenes as well as their derivatives. Particularly preferred are monofunctional, di-, tri- and polyfunctional(meth) acrylic esters and urethane (meth)acrylic esters listed below.
a. Monofunctional(meth)acrylates
   Methyl(meth)acrylate, n- or i-propyl(meth)acrylate, n-, i- or tert.-butyl(meth)acrylate and 2-hydroxyethyl-(meth)acrylate
b. Difunctional (meth)acrylates
   Compounds of the general formula: 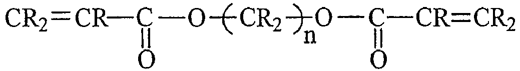 in which each R independently is hydrogen or methyl and n is a positive integer from 3 to 20, such as, for example, di(meth)-acrylate of propanediol, butanediol, hexanediol, octanediol, nonanediol, decanediol and eicosanediol, compounds of the general formula: 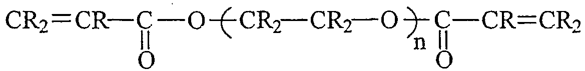 in which each R independently is hydrogen or methyl and n is a positive integer from 1-14, such as, for example, di(meth)acrylate of ethylene glycol, diethylen glycol, triethylene glycol, tetraethylene glycol, dodecaethylene glycol, tetradecaethylene glycol, propylene glycol, dipropylene glycol and tetradecapropylene glycol; and glycerine di(meth)acrylate, 2-2'-bis[p-(γ-methacryloxy-β-hydroxypropoxy)phenylpropane], or bis-GMA, bisphenol A dimethacrylate, neopentyl glycol di(meth)-acrylate, 2,2'-di(4-methacryloxypolyethoxyphenyl)propane having 2-10 ethoxy groups per molecule and 1,2-bis(3-methacryloxy-2-hydroxypropoxy)butane.
c. Trifunctional or multifunctional(meth)acrylates Trimethylolpropane tri(meth)acrylates and penta erythritol tetra(meth)acrylate.
d. Urethane(meth)acrylate
   Conversion products of 2 mole (meth)acrylate monomer containing hydroxyl groups with one mole diisocyanate and conversion products of a urethane prepolymer
   comprising two N-C=O terminal groups with a methacrylic monomer comprising a hydroxyl group such as are represented, for example, by the following general formula: 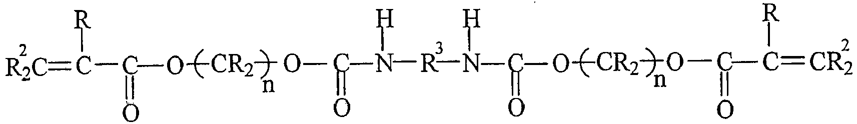 in which each R independently signifies hydrogen or methyl, and each n is independently a positive integer from 3 to 20, each R² represents an organic residue, preferably alkyl groups or hydrogen, and R³ represents an alkylene group, preferably methylene, ethylene or propylene.
e. silica- or silicone-based acrylates
   Also silica- or silicone-based mono-, di- or multiacrylates are suitable.

The stated monomers are used either alone or as a mixture of two or more monomers. Monomers that are preferably used in the dental material according to the invention include, above all, 2,2-bis-4(3-methacryloxy-2-hydroxypropoxy)-phenylpropane (bis-GMA),3,6-dioxaoctamethylene dimeth-acrylate (TEDMA) and/or 7,7,9-trimethyl-4-13-di oxo-3,14-di oxa-5-12-di aza-hexadecane-1-16-di oxymethacrylate (UDMA).

The monomers also can contain basic groups (e. g. amines) or also acidic groups as described in US 4,806,381.

Depending on the type of catalyst employed, the dental material may be polymerizable at room temperature or at elevated temperatures and/or may be polymerizable by means of light. As catalysts for heat polymerization, use may be made of the known peroxides such as dibenzoyl peroxide, dilauroyl peroxide, tert.-butyl peroctoate or tert.-butyl perbenzoate. α,α'-bis(isobutyroethyl ester), benzopinacol and 2,2'-dimethylbenzopinacol are also suitable.
As catalysts for photo polymerization, use may be made, for example, of benzophenone and its derivatives, as well as benzoin and its derivatives. Other preferred photosensitizers are: α-diketones, such as 9,10-phenanthrenequinone, diacetyl, furil, anisil, 4,4'-dichlorbenzil and 4,4'-dialkoxybenzil or camphorquinone. Use of the photosensitizers together with a reducing agent is preferred. Examples of reducing agents are amines auch as cyanoethyl methylaniline, dimethylaminoethyl methacrylate, triethyl amine, triethanol amine, N,N'-di methyl-sym.-xylidine and N,N-3, 5-tetramethylaniline and 4-dimethylaminoethyl benzoate.

As catalysts for polymerization at room temperature, use may be made of systems that supply radicals, for example benzoyl peroxides or lauroyl peroxide, together with amines such as N,N-dimethyl-sym.-xylidine or N,N-dimethyl-p-toluidine. Use may also be made of dual curing systems as catalysis, for example photoinitiators with amines and peroxides. As photocatalysts, mixtures of catalysts that cure in UV light and catalysts that cure within the range of visible light can also be taken into consideration. The quantity of these catalysts in the dental material customarily amounts to from 0.01 to 5 wt-%.

Another possible curable matrix according to this invention is one containing ring-opening monomers, polymerizahle by ring-opening mechanism or polyaddition. Such polymerisable materials are called epoxides and could contain, as an example, an oxiranring:

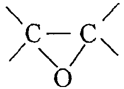

Polymerisable epoxides could be monomers or polymeric epoxides and they could be aliphatic, cyclo-aliphatic, aromatic or hetero-cyclic. Such epoxides are described e. g. in EP 1141094 B1 and EP 1117367 B1. These materials in general contain on average one polymerisable epoxide group per molecule. More preferably they should contain at least 1,5 polymerisable epoxide groups per molecule. The polymerisable epoxide could be a linear polymer with an epoxide endgroup (diglycidyl ether of polyoxyalkylene glycols), a polymer with oxiran-groups in the bulk of the molecule, (polybutadien polyepoxide) and epoxy group in a side chain of the polymer (glycidyl methacrylate polymer or co-polymer). These epoxides could be pure or mixed with one, two or more epoxide groups per molecule.

Suitable epoxide materials could contain cyclohexenoxide groups, such as epoxycyclohexane carboxylate, with typical examples: 3,4-epoxy cyclohexyl metyl-3,4-epoxy cyclohexane carboxylate, 3,4-epoxy-2-metylcyclo-hexylmethyl-3,4-epoxy-2-methylcyclo hexane carboxylate and bis-(3,4,epoxy-6 methyl cyclohexyl methyl) adipate (for a detailed description see also US-Pat.-No. 3, 117, 099).

Further suitable epoxides are silicones with epoxy-functionality, especially cyclohexylepoxy groups with the silicone in the bulk of the molecule. Commercially available materials belonging to this group are UV 9300, UV 9315, UV 9400 and UV 9425 from GE Bayer Silicones.

Especially suitable and biocompatible are monomers with epoxides and organic backbones (linear or cyclic), as described in DE 19860361 and WO 00/19967 with the required starter systems for light-, heat- or cold-polymerisation. Hydroxy-containing materials or vinyl ethers could also be added to the epoxide-resin.

A further ring opening monomer could also contain derivatives of the ortho-carbonic acid (as an example the general structure of spiro-ortho carbonate is shown, Beyerley et al. US 5,556,896):

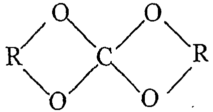

Also suitable are ormocer-matrices, such as described in WO 92/16571, DE 4133494 or DE 10016324.

A newer group of polymerisable monomers, suitable for restorative purposes, are those based on silicone monomers. Examples of this group could be resin matrices as described in DE 3915592 Al for use as dental cements. The dental cements comprised a silicone oil modified with carboxylic groups as curable liquid and a metal oxide and/or metal hydroxide as accelerator.

Another suitable example of silicone-based resin matrix has been described in US 3,127,363. The matrix is formed by the condensation Polymerisation of an organosilioxane A (of the general formula XO-Si(R)₂-[O-SiR₂]ₙ-O-Si(R)₂-OX with a trifunctional cross-linking agent B (for example one of the general formula RSi(OH)₃).

A two-component resin matrix based on vinylsilicone oils can also be prepared. Component l contains one or more silicone oils with at least one Si-H group, while component II contains one or more silicone oils with at least two vinyl groups. In addition, either component contains a catalyst for their curing. In this way, as described in EP.0864 312 Bl, a root canal filler but with addition of nano apatite capable of transporting fluoride or whole nano apatite crystals into finest dentinal tubulis, can be prepared. Addition of component II is not a prerequisite. It is as well possible to get a slow hardening material using only a component I, having hydrolysis of the SiH-group to SiOH, and than recticulating SiOH with other SiH. This system, filled with nanao apatite, can be used as an adhesive sealant similar to those described in WO 0211681.

Another important group related to this invention are materials wherein the hardenable matrix is based on cements (acids crosslinkable with basic components). Classical cements used in the dental or medical field mainly consist of acids and reactive alkaline glasses or metaloxides. The term "classical" is used to denote cements free of resins. Resin-modified versions of these cements are readily prepared by incorporation of polymerizable resins. Cements comprise a number of distinct groups:

The "classical" dental polyalkenoate cement comprises finely ground fluoro-aluminosilicate glass powder (or basic oxides), polyalkenoic acid(s) and water. The formation of the water-insoluble dental polyalkenoate cement from this water-based formulation takes place over a number of steps and over a period of time. In summary, in the presence of water, the polyalkenoic acid(s) attack the fluoro-aluminosilicate powder at their acid-susceptible sites and as a result liberate ions (especially alkaline earth metal ions and aluminum ions). These liberated metal ions undergo ionic bonding to alkenoic groups of the polyalkenoic acid(s) to form a crosslinking structure. In this way the cement "salt" is gelled into a water-insoluble hard mass of material. The realization of this water-insoluble set polyalkenoate cement takes place rapidly over the initial several minutes from the start of the cement mix and then slower over several further hours and days. During this time the cement is susceptible to saliva attack.

For example suitable crosslinking acids of the polyalkenoate cements can be polyacrylic acid, polyitaconic acid, polymaleic acid, lactic acid, polyvinyl sulphonic acid, polystyrene sulphonic acid, polysulphoric acid, polyvinyl phosphonic acid and polyvinylphosphoric acid or copolymers of these. For example the basic components of the polyalkenoate cements can comprise fluoro-aluminosilicate glass powders which have e.g. as the main composition from 10 to 25 % by weight of Al, from 5 to 30 % by weight of Si from l to 30 % by weight of F, from 0 to 20 % by weight of Sr, from 0 to 20 % by weight of Ca, and from 0 to 10 % by weight of alkali metal ions (e.g., Na⁺, K⁺ etc.), based on the whole weight of the glass and which are prepared by mixing raw materials containing these components and melting the mixture, and then cooling and pulverization so as to have a mean particle size of from about 0.2 to 20 µm.

Of the basic oxide variant of water-based polyalkenoate cements, preferred is the zinc oxide and deactivated zinc oxide/magnesium oxides of the zinc polycarboxylate cements. Preferred also are the newer zinc-based dental compositions [European Patent no. EP0883586, 1997]. Other basic oxide cements include those prepared from Be, Cu, Mg, Ca, Sr and Ba and combinations thereof.

The "classical" dental silicate cement comprises finely ground fluoro-aluminosilicate glass powder (or basic oxides) similar to those used in polyalkenoate cements, phosporic acid and water.

The "classical" dental phosphate cement comprises finely ground modified zinc oxide powder (or basic oxides), phosporic acid and water.

Other classical dental cements can be as well cements mostly consisting of zinc oxides or other metaloxides and weak organic acids or phenol-type compounds (e.g. eugenol).

The dental cement composition can be in a powder, liquid or paste format. In addition to the dental cement compositions according to the present invention, known ultraviolet light absorbers, plasticizers, antioxidants, bactericides, surfactants, etc. can be added, if desired.

As important component in the above mentioned hardenable matrices or cement systems, the present invention specifically uses nanostructured apatite of the general formula:

Ca₁₀(PO₄)₆A_{z}(OH)_{2-z}

wherein A is F⁻ wherein the particle size of at least 50 wt.-% of the apatite particles is in the range from 2 to 200 nm, and wherein z=0 (Hydroxyapatite) or z=2 (Fluoroapatite).

It is known, that apatites are involved in the main inorganic process of calcification of normal tissue (e. g. enamel, dentine, cement, bone) and they are found associated with other phosphatic and non-phosphatic minerals in pathological calcifications. The main one of these compounds is hydroxyapatite (or hydroxylapatite, HA), having the stoichiometric formula Ca₁₀(PO₄)₆(OH)₂ (or Ca₅(PO₄)₃OH), and being in its synthetic (biocompatible) form, it is the apatite material which is most widely exploited at a commercial level for several indications in dentistry, orthopedics and maxillofacial surgery. However, HA is never found in a pure state in the biological tissues. This is due to the possible isomorphous replacements of the ca²⁺, PO₄³⁻ and OH⁻ ions. The calcium ion generally (but not exclusively) may be partially replaced by a number of cations having oxidation number +2; the phosphate ion (site B) may be replaced by carbonate, acid phosphate, pyrophosphate, sulphate, aluminate and silicate ions, and the hydroxyl ion (site A) may be replaced by halogenide, carbonate and oxide ions.

Amongst the possible phosphates and apatite materials, either naturally-occuring or synthetic apatites are suitable for the production of materials for the use in the odontostomatologic and biomedic field; hydroxyapatite has already been presented as the most widespread material. In its structure, the phosphate and calcium ions are placed approximately according to a hexagonal prism; in the direction of the elongation (crystallographic axis c) the prism is crossed by a channel having a diameter of 3-3.5 Å, housing any OH- groups or other possible substituting ions (e. g. fluorine and chlorine. HA may crystallise in two forms: a monocline form (spatial group P2₁/b) and a hexagonal form (spatial group P6₃/m). In the monocline form, a binary symmetry axis is present along the axis c, while in the hexagonal form the symmetry axis becomes hexagonal. Concerning the possible cation replacement in the apatite formula, although this problem has been studied in the literature, the only information of some interest is that the solid solutions are structurally more ordered when the size of the replacing cation is large. It is not possible to theoretically forsee the ability of calcium to be replaced by chemically and crystallographically similar ions. In addition, also when this is the case, the extent of the isomorphous replacement may be partial. However, it is ascertained that the preparation method has a substantial influence on the extent of the replacement. Possible cations that can substitute into the HA lattice are: Na⁺, K⁺, Mg²⁺, Sr²⁺, Ba²⁺, Y²⁺, T1²⁺, Zr²⁺, Mn²⁺, Fe²⁺, Pd ²⁺, Cu²⁺, Ag⁺, Zn²⁺, Sn²⁺, Re²⁺, Re³⁺, Al³⁺ In³⁺ and/or Y³⁺.

Especially preferred in this invention are Sr, Ba, Zn and Y as substitutes in the apatite.

The presence of strontium in apatites for odontostomatological use is considered to be important in connection with a possible cariostatic effect thereof (in addition to the effect of reduction of the dentine sensitivity), and confers a lower solubility and a higher resistance to thermal treatments on the apatite. In addition, the radiopacity is increased.
As pointed out before, the basic structure of hydroxyl apatite may also be substituted with anionic groups. In particualar, in carbonat-apatite the carbonate ion may replace the hydroxyl ion (site A) or the phosphate ion (site B) or both. Although the replacement of the carbonate ion in the site B is preferred in biological samples, it is also possible to obtain carbonate-apatites of the type A synthetically, by means of high temperature reactions. On the other hand, carbonate-apatites of the type B or mixed type A+B are mainly obtained by precipitation from solutions.

The presence of the carbonate ion in the different sites, which is hardly detectable by x-ray diffractometry, may be evidenced through IR-spectroscopy, since the position of the absorbtion bands of the carbonate ion directly depends on the occupied site. The presence of the hydroxyapatite modifies the lattice dimensions: if the said ion occupies the A-site an increase in the a parameter is obtained, as a consequence of the greater size of the CO₃²⁻ ion with respect to the OH⁻ ion; if, on the contrary, the carbonate ion occupies the B site the same parameter undergoes a contraction, due to the smaller 0-0 distance in the CO₃²⁻ ion with respect to the PO₄³⁻ ion. In addition, it has been observed that the inclusion of the carbonate ion in apatite results in a reduction and a change in morphology of the crystals, which appear to change their shape from a needle-like shape of comparable length in the various crystal dimensions. The solubility increases as well, while the thermal stability decreases.

In fluoro- and chloroapatites the F⁻ and Cl⁻ ions, respectively, replace the hydroxy ion; the said replacement, theoretically, may be total. Fluorapatite is characterised by an increase in the crystal dimensions, by a decrease in the A Parameter of the unit cell, by a lower solubility and by an enhanced thermal stability. The lower solubility, and therefore the higher lattice stability of the fluorapatites substantiates the present use of the fluoride ion in the therapy of bone affections and of dental caries.

Preferred is a dental composition as defined above wherein x, y and z are 0 (Hydroxyapatite).

Moreover preferred is a dental composition as defined above wherein x and y are 0, A = F⁻ and z = 2 (Fluorapatite).

The nano-structured apatites may be produced by any one of the several known methods already in use for the production of nanocrystalline materials, such as the synthesis methods from atomic or molecular precursors (e. g., chemical or physical vapour deposition, condensation in gas, chemical precipitation, reations from aerosol), or the methods of production from mass precursors (e. g. by mechanical attrition, by crystallisation from the amorphous state, by phase separation).

Several new opportunities also exist in the field of the production of nanophasic materials assembled from atomic clusters synthesised through physical and chemical methods. For instance, chemical precipitation represents one of the conventional methods for synthesising ultrafine powders or colloidal suspensions that has been successfully applied in the synthesis of nanometer-sized clusters with narrow dimensional distribution, for instance by applying the sol-gel technique or the inverse micelle method. Also, many high-temperature gas reaction methods are presently available for the synthesis of nanometric clusters, or of nanostructured powders of bigger size (R. W. Siegel, 1991, loc. cit.).

Nano crystalline materials are in general synthetically produced as inter-connected phases or in granular form and are charcterised by having length less than 200 nm. Depending of the number of dimensions these nano-crysatlline materials could be classified (as defined in R. W. Siegel, Materials, Science and Technology, vol. 15: Processing of Metals and Alloys, R. W. Chan, 583 (1991).

The specific properties of nano crystalline materials results from three characteristic features: (i) the small size of less than 200 nm, (ii) the large percentage of particle-sharing at grain interface and (iii) the interaction between the single particles. The nano particles have a high surface : volume ratio. In a material with average particle size between 10 - 15 nm there are 15 - 50% of atoms being shared at grain interfaces. The number of grain interfaces in nano crystalline materials are very high compared to conventional materials. This number, directly linked to the particle size, controls the interaction between the particles and the biological environment. The control of particle size during the synthesis of nano apatite directly influences this biological interaction. The interaction between nano-apatite in a polymerised matrix and its biological environment is much more intenset than that obtained using normal sized apatite in this matrix. Therefore, in this invention nano apatite based materials have been found to be useful as bone cements, bone filling materials and especially tooth-substitutes.

Due to their very small size, another important property of the nano apatite particles is their non-interference with light of the wavelength from 400 nm to 900 nm and they also do not interfere with light of longer wavelengths. Therefore, due to this invention, fillers such as apatite with usually unsuitable refraction indices of about 1,58 to about 1,64 and in normal resin matrices of about 1,45 to about 1,54, which result normally in white-opaque mixtures and white-opaque hardened solids, now lead, in the case of nano-particle sizes, to nearly transparent mixtures which are useful for dental esthetic applications.

According to the invention, the sizes of the nano-particles of the apatite in biomaterials should be in the majority within the range from 2 to 200 nm, and even more preferably within the range from 2 to 90 nm. Especially preferred is the range from 10 to 70 nm.

In a preferred version of this invention the nano apatite particles are surface treated to improve their incorporation into the requisite matrix, thereby improving the mechanical properties of the resulting cured materials.

For example, the apatite fillers can be surface treated with mono- or multi functional methacrylated esters of phosphoric, phosphonic, carboxylic- or combinations thereof. In particular, the esters of hydroxyethyl methacrylates and of glycerine dimethacrylates are preferred.

Also, polymethacrylated polyvinylphosphoric acids, or polyvinylphophonic acids or polycarbonic acids can be used for surface modifications. In addition, reactants such as phosphoric acid esters, phosphonic acid esters and carboxylic acid esters with vinyl groups are useful for this surface treatment. Also maleic acid can be used. The choice of surface treatment reactant depends on the pendant group contained on the reactant, e. g. methacrylate pendant groups are suitable for methacrylate-based resins, vinylether pendant groups or epoxides are suitable for epoxide-based resins, vinyl- or SiH- pendant groups are suitable for vinylsiloxane-based matrices. Of course, the purpose of this surface treatments is to create an organic-surface treatment on the inorganic fillers in order to improve their incorporation into organic matrices via covalent bondings, with the result of improved mechanical properties of the cured material.

Another objective of surface treatment can be to increase the dispersivity and thereby the filler-load content of apatite in materials via inorganic surface treatment of the apatite, e. g. with phosphates or non-organic siloxanes.

A further surface treatment method is the application of a layer of SiO₂ or ZrO₂ in a nanometer scale followed by a treatment of a functional silane like methacryl-, glycidyl-, amino- or vinylorganosilane, as far as the ionexchange properties are not too much influenced.

The nano-apatite filler can also be incorporated into a relevant material (resin matrix, or cements), hardened, ground and then used as a filler, with or without the above-mentioned surface treatments.

The quantity of nano apatite incorporated into the material of this invention must be sufficient to ensure adequate ion exchange with the oral environment while maintaining good mechanical properties. With this in mind, the weight-percentage of an apatite filler should be between l and 70 % of the total cured material. Depending on the application, for good mechanical properties the apatite filler content is preferably 1 - 30 % and for high-load bearing areas materials the apatite filler content is preferably 1 - 15 %.

Besides the described essential and characteristic nano apatite filler component, the hardenable materials according to this invention can also optionally contain other fillers.

Further fillers can be ground powders of SiO₂ and/or glass ceramics and/or glasses, and/or micro-spheres of an average particle size of 0,2 to 40,0 µm and with a refraction index of 1,45 to 1,54. Other fillers could be inorganic and/or organic fibers, other nano-fillers such as SiO₂ and metal oxides in nano size, agglomerated nano-fillers, unfilled or filled pre-polymerised materials, ion released glasses or reaction products of these. Other ground powders are Ba-, Sr- or Li-Al-silicate glasses with an average particle size in the range of 0,2 to 2,0 µm and refraction indices of 1,48 to 1,53. These fillers can be chosen to impact special desirable properties, such as increased radiopacity or release of ions (biological or drug release).

Prefered micro-spheres are fillers described in DE-A 32 47 800. The average particle size is in the range of 0,1 to 1,0 µm in particular in the range of 0,15 to 0,5 µm.

Inorganic or organic fibers that can be used include fibers of glass, alumina oxide, polyethylene oxide or carbon, cabable of directionally strengthening the fiber-reinforced material. Other nano-fillers, e. g. agglomerated nano-fillers based on SiO₂ (coated with metal oxides, or with metal oxide incorporation), are polydispersed or monodispersed or mixtures of poly- and mono-dispersed spheres (W. Stöber et al. in J. Colloid and Interface Science 26,62(1968) and 30, 568 (1969), US-Patent 3,634,588, EP 0 216 278 and EP 0 275 688).

Optionally, sintered fillers, as described in EP 0 113 926 can be co-incorporated.

If desired, other fillers can be added, for example to increase radiopacity (as described DE-OS 35 02 594) provided that such fillers are preferably less than 5,0 µm in particle size.

In any case, these fillers can be surface treated in a manner similar to that of the nano apatite filler to improve their incorporation in materials, thereby improving the mechanical properties of the cured material.

In order to attain a certain desired viscosity, small amounts of silanized microfine powders or pyrogenic or precipitated silicic acid can be incorporated into the dental material, preferably in quantities of less than 50 wt.-% (of the dental material). More preferable, the quantity should be between l to 25 wt.-%, most preferably between 3-10 wt.-%.

Other possible filler materials are bioactive or antibiotical substances, as described in other publications, e. g. WO 97/17285.

The dental materials according to the invention can be used intraorally in direct contact to saliva and in the more visible areas because of their excellent optical properties.

The apatite component of these materials can release and absorb ions (i. e, fluoride, phosphate, calcium, etc.) to the surrounding enamel and saliva. The ion absorbtion can be induced externally, for example from tooth paste and release and absorbtion are reversible.

The desired purpose of this invention, namely the ion exchange between the apatite-containing dental material and the biological environment on the one hand and high translucency and hardness of the bioactive material on the other, are possible with the incorporation of nano apatite fillers in polymerisable systems and in cements.

The nano-apatite additives according to the invention in dental formulations of low filler content resins, dental resin composites, ormoceres, gionomers, compomers, resin reinforced cements, classical cements (e.g. classical polyalkenoate cements, silicate cements, phospate cements), oxirane-, silorane- and silicone-formulations can be a part of tooth fillings, inlays, onlays, adhesives, cements, coating materials for crowns and bridges, materials for artifical teeth, parts of it or veneers, dentin - and enamel bondings, liners, core build-up materials, sealants, root canal filling materials or other curing materials for the prosthetic, conservative and preventive dentistry.

As a bone cement or bone filling material there are possibilities for a better biological interaction between natural substance and artifical substitute, improved biological ion exchange and a higher mechanical strength.

The present invention is illustrated by the following examples:

### Example 1: Preparation of nano-crystalinic Ca-fluorapatite

Nano-crystalinic fluorapatite was crystalized from ternary micro-emulsion containing Empilan KB6ZA (ethoxylated laurylalcohol, Albright & Wilson, Meuse, France) as non-ionic surfactant, octane as oil phase (Sigma-Aldrich, Schnelldorf, Germany) and a 1.0 M CaCl₂ aqueous solution as aqueous phase. The three components were mixed under vigorous magnetic stirring. Three microemulsion compositions with a fixed surfactant to octane ratio of 3:7 at 30 °C (samples I-III), containing 30 (I), 36.36 (II) and 50 (III) wt-% 1.0 M CaCl₂ aqeous solution were chosen for preparing the fluorapatite powders. To these solutions a stoichiometric amount of a 0,6 M Na₂HPO₄ / 0,2 M KF aqueous solution was added, again under vigorous stirring. The mixtures were set aside at 30 °C for 24 h. The powder was separated by washing away the surfactant and oil phase using distilled ethanol and centrifugation. The precipate was washed twice with ethanol and once with distilled water. Finally the derived fluorapatite powders were freeze-dried for 48 h. The extremely fine powders were analysed for their crystallinity, morphology and particle sizes by XRD and EDX. High resolution TEM-pictures showed well-defined rod-like crystals. The particle sizes were between 20 and 130 nm. X-ray diffractometric diagrams showed a high value of crystallinity.

### Particle sizes

**Table 1: Diameter and length of the papatite crystals (Sample I-III) of the TEM-pictures.**

| Sample | diameter [nm] | length [nm] |
|---|---|---|
| I | 28 | 84 |
| II | 29 | 127 |
| III | 23 | 52 |

Pictures 1-3 are the TEM-pictures of the flourapatites of samples I-III.

Picture 4 shows the XRD-pattern of samples I-III.

For comparison, Picture 5 shows the XRD-diagram of an amorphous calciumphosphate.

### EDX-Data of nano-apatite

SEM-EDX data of samples I - III (energy-dispersive X-ray spectrometry of the powders) corresponded to the expected values for calciumfluorapatite:

**Table 2: SEM-EDX data of calcium fluor apatite.**

| Sample | Mol % O | Mol % F | Mol % P | Mol % Ca | Ca/P Mole ratio |
|---|---|---|---|---|---|
| Ca-F-Apatit (calculated) | 57,1 | 4,8 | 14,3 | 23,8 | 1,66 |
| I | 60,64 | 5,41 | 13,67 | 20,28 | 1,48 |
| II | 59,79 | 5,67 | 14,26 | 20,28 | 1,42 |
| III | 56,87 | 4,96 | 15,40 | 22,77 | 1,48 |

### IR-Spectrometry of nano-apatite

All three samples showed the characteristic wave numbers of calcium fluorapatite.

### IR-wave numbers [cm⁻¹]

3426 / OH stretch , 1638 w / H₂O crystal water, 1099vs / u₃ PO₄ antisym.,1038 vs / u₃ PO₄ antisym., 965 w / u₁ PO₄ sym., 868 w / CO₃ stretch, 606 s / u₄ PO₄, 567 s / u₄ PO₄, 474 w / u₂ PO₄, 326 s / u₃ Ca₃-F "sublatticemode", 273 / u₃ Ca-PO₄ "lattice mode", 229 / u₃ Ca-PO₄ "lattice mode"
FT-IR data of Ca₁₀(PO₄)₆F₂.
Intensities: vs = very strong, s = strong , m = medium), w = weak

### Example 2: Surface modification of a nano apatite powder

100 g of the nano-apatite powder of sample I was made into a slurry with aceton and treated with 6 g Hydroxyethylphosphoric acid ester under stirring. After 2 hours stirring, centrifugating and washing three times with aceton the obtained powder was dried.

### Example 3: Preparation of resin-composites with nano apatite filler

Different resin-composites with nano apatite were prepared with the apatite powder of example 2. Thereby, a resin-matrix based on light curing dimethacrylates was prepared with different amounts of fillers (see more down). Specimens of the obtained pastes were prepared, light cured with a Dentacolor XS curing apparatus (Kulzer, Deuschland) and their different properties were examined.

The resin-matrix was prepared as follows:
10 parts triethylene glycole dimethacrylate
10 parts Bis-GMA.
10 parts urethane dimethacrylate
0,05 parts camphorchinone
0,05 parts dimethyl aminoethylmethacrylate

In the present application parts referred to parts by weight unless indicated otherwise.

20 parts of the resin matrix were filled with 2 parts of Aerosil 202 and the following filler was worked in additionally:

### FAP 0 (reference example)

72 parts barium aluminiumborsilicate glass 0,6 µ, methacryl-silanized
0 parts nano-fluorapatite of example 1 (I), modified as described in example 2

### FAP 4

72 parts barium aluminiumborsilicate glass 0,6 µ, methacryl-silanized
4 parts nano-fluorapatite of example 1 (I), modified as described in example 2

### FAP 8

68 parts barium aluminiumborsilicate glass 0,6 µ, methacrylsilanized
8 parts nano-fluorapatite of example 1 (I), modified as described in example 2

### FAP 16

60 parts barium aluminiumborsilicate glass 0,6 µ, methacrylsilanized
16 parts nano-fluorapatite of example 1 (I), modified as described in example 2

### FAP 30

47 parts barium aluminiumborsilicate glass 0,6 µ, methacrylsilanized
30 parts nano-fluorapatite of example 1 (I), modified as described in example 2

Picture 6 is a TEM-picture of FAP 30.

### FAP 39

37 parts barium aluminiumborsilicate glass 0,6 µ, methacrylsilanized
39 parts nano-fluorapatite of example 1 (I), modified as described in example 2

### FAP 48

27 parts barium aluminiumborsilicate glass 0,6 µ, methacrylsilanized
48 parts nano-fluorapatite of example 1 (I), modified as described in example 2

### FAP 65

0 parts barium aluminiumborsilicate glass 0,6 µ, methacrylsilanized
65 parts nano-fluorapatite of example 1 (I), modified as described in example 2

### Physical data of nano-apatite-composites

**Table 3: Some physical properties of the nano-apatite-composites**

| | FAP 0 (comparison) | FAP 4 | FAP 8 | FAP 16 | FAP 30 | FAP 39 | FAP 48 | FAP 65 |
|---|---|---|---|---|---|---|---|---|
| Depth of cure* [mm], 30sec | 6,0 | 5,5 | 5,7 | 5,8 | 6,1 | 5,9 | 5,7 | 5,2 |
| Opacity [%] | 75,0 | 74,6 | 80,9 | 85,5 | 83,8 | 83,7 | 84,5 | 79,0 |
| Flexural strength [MPa] | 165 | 150 | 128 | 154 | 104 | 84 | 94 | 67 |
| Elasticity modulus [Gpa] | 13,6 | 12,2 | 13,7 | 13,1 | 10,3 | 8,2 | 8,8 | 5,9 |
| Diametrale Tensile strength [MPa] | 49,0 | 45,8 | 45, 9 | 36,2 | 29,2 | 29,2 | 23,1 | 14,9 |
| Barcol hardness | 86 | 87 | 86 | 85 | 87 | 88 | 87 | 78 |
| Solubility Mg/mm³ | 0,24 | 0,32 | 0,18 | 0,33 | 0,39 | 2,37 | 6,31 | 38,65 |

### Conclusions:

1. The depth of cure is adequate and comparable with commercially available composites (as shown for FAP 0)
2. The opacity is good enough for the preparation of esthetic restorative materials and is comparable with commercially available tooth filing materials (as a comparison: a filled paste of type FAP 65 with 12 µ fluorapatite has a chalky white color and an opacity of 99 %),
3. The flexural strength for highly loaded areas, such as occlusal surfaces or loaded bone areas is sufficient up to filler value FAP 30, for under fillings and bone fillings even up to filler values of FAP 65 (only nanoapatite),
4. The modulus of elasticity is in the range of commercially availbale tooth filling materials and bone cements (ca. 3 - 14 GPa),
5. The diametral tensile strength is "good to sufficient", depending on the application (as described under 3.)
6. The barcol hardness is slightly increased in comparison to composites without nans-apatite
7. The water solubility is "good to acceptable" up to filler values of FAP 50, for under filling areas also up to values of FAP 65.

The important property of the exchange of ions, important for bioactive action, could be demonstrated by measuring fluoride ion release and fluoride ions recharge, here described with help of the composite examples FAP 30 - FAP 65.
For this, 2 specimens with diameter of 2 cm and thickness of 2 mm were placed in distilled water. Fluoride release was measured (see table 4). After three weeks the fluoride release had dropped to negligible amounts. After this, the specimens were stored for three days in a 10-wt% NaF-solution, thoroughly washed three times by storage for 4 hours in water. Fluoride release was measured, again there was negligible release after three weeks. This procedure was repeated once again. It could be shown (see table 4) that:
1. The flouride release and fluoride recharge and the rates of these two phenomena were found to be reversible.
2. The amount and the rate of the fluoride release is comparable to the rate exhibited by light-curing glass ionomer-cements (in this case Fuji II LC, GC Corp. Japan). These glass-ionomer cements are also fluoride rechargeable, but in contrast to the described one component pastes these cements are two-component powder-liquid systems.
3. The rate of fluoride release is higher than in compomers (in this case Dyract, Dentsply), which is a one- component light-curing glass ionomer cement in paste-form
4. The formulation without nano-apatite (FAP 0), which represents the state of the art of composites, showed a negligible rate of fluoride release and fluoride rechargeability.

### Fluoride release

**Table 4: Maximal flouride releasing rate**

| | Composite | Compomere | Composite with nano-apatite | | | | Resin-reinforced cement |
|---|---|---|---|---|---|---|---|
| | FAP 0 | Dyract * | FAP 30 | FAP 39 | FAP 48 | FAP 65 | Fuji II ** |
| | | | | | | | |
| Initial | 0,002 | 0,065 | 0,072 | 0,072 | 0,093 | 0,272 | 0,484 |
| After first fluoride recharge | 0,004 | 0,044 | 0.112 | 0,154 | 0,116 | 0,297 | 0,379 |
| After second Fluoride recharge | 0,003 | 0,056 | 0,142 | 0,159 | 0,131 | 0,379 | 0, 688 |

| | | | | | | | |
|---|---|---|---|---|---|---|---|
| * manufactured by Dentsply, Konstanz, Germany ** manufactured by GC corporation, Japan | | | | | | | |

### Example 4: Preparation of a heat cure resin filled with Nano-apatite for manufacturing of pre-shaped veneers

Heat cure resins were produced by preparing a variety of powder mixtures:
6 parts of Colacryl D 150 (Lucite, GB)
3 parts of Plex 6690 F (Rhöm, Germany)
1 part of MW332 (Rhöm)
0,1 part of benzoylperoxide
x parts of nano-fluorapatite of example 1 (I), modified as described in example 2,
where x means 0, 0.2, 0.6 or 1 part,
and kneading the different powders to heavy consistency pastes with a mixture of
6,7 parts of methylmethacrylate
7,3 parts of tetraethylenglycoldimethacrylate

The pastes were filled into a veneer mould, heat cured for 15 min under pressure of 30 bar at a temperature of 120 C°, cooled down and released from the mould.

### Results:

The translucency of the veneers were good and the apparence was enamel-like. Since the main problem of veneers, cured under these conditions, is lower capability of getting strong adherence by adhesives to the tooth surface because of ist high conversion of double bonds and low swellability by adhesive resins, the problem of adhering could unforeseeable be improved by incorporating nano apatite into the veneers. Adhesivenes was determined by measuring shear strength between cured cylinder of veneer material and dentin, adhered with selfetching dentinoenamel adhesive Revolcin One (Merz, Germany) on both sides, veneer and dentin, and with flowable composite Starflow (Danville, USA) inbetween and light cured for 20 sec with dental curing light Translux (Kulzer, Germany). Adhesivenes of the samples is measured after 24 h storage in water at 37 C° (results see table 5).

**Table 5**

| Veneer filled with nano-apatite | 0% | 2% | 6% | 10% |
|---|---|---|---|---|
| Adhesivenes to dentin (Shear strength N/mm²) | 6,5 | 9,7 | 10,1 | 21,4 |

The results indicate better ability of getting adhered to tooth when the veneers are filled with nano apatite when using selfetching adhesive Revolcin One.

### Example 5: Addition of Nano-Apatite into a selfetching dentinoenamel adhesive

Into Part B of the dentinoenamel adhesive APOL SE BOND (BFC Dentaire, France) either 10% of the nano-fluorapatite of example 1 (I), modified as described in example 2. (herein called "Part B 10%APA") or Part B which is left unchanged is incorporated. For the adhesive tests, one part Part A of the APOL SE BOND was either mixed with one part PART B 10%APA or with one part unmodified Part B, applied onto an even dentine as well as onto an even enamel surface according the instruction of the manufacturer and adhered to it in the form of a cylinder of light cure composite APOLCOMP (BFC Dentaire) by light curing the composite in that cylinder matrix by 20 sec Translux light. Adhesivenes of the samples was measured as shear strength (MPa/mm²) after 24 h storage in water at 37 C° (results see table 6).

**Table 6**

| | Part A + Part B | Part A + Part B 10%APA |
|---|---|---|
| Adhesivenes on dentine | 6,6 | 9,4 |
| Adhesivenes on enamel | 18, 9 | 26,1 |

As to be seen, the addition of nano fluorapatite fillers to a dentinoenamel adhesive is able to enhance the adhesivenes of the system dramatically. This was not foreseeable. In the contrary, normally addition of fluorides into a bonding agent lowers bond strength. It is important that addition of nano apatite with its ability of fluoride- and calcium- release can be incorporated into the adhesive without disturbing the adhesive strength, in contrary to enhance it.

### Example 6: Preparation of a composite formulation based on ring-opening monomers with Nano-apatite

As an example of a composition with ring-opening monomers filled with nano apatite, the following flowable paste was prepared:
21 parts Silbione UV Polymer 30 (Rhodia)
2 parts Silbione UV Polymer 30 Photosensibilisator (Rhodia)
1 part Rhodorsil Photoinitiator 2074
16 Parts nano-fluorapatit of example 1, modified as described in
example 2

The occlusal area of a molar tooth was etched with a commercially available etching gel, containing 37% phosphoric acid, rinsed with water, and treated with an alcohol- and carboxilic groups-containing adhesive ("Flowsive 2", R-Dental, Germany).
After this, the above-mentioned mixture of example 4 was coated on the fissures of the molar tooth and polymerized for 40 seconds with a dental halogen light. The layer of the mixture can protect the fissures not only mechanical, but also through fluoride-ions, which could be recharged through tooth brushing.

### Example 7: Preparation of nano apatite containing Classical cements

As an example for a classical cement the following compositions were prepared:
4.1. Glass-ionomer cement for luting (comparison example)
9 parts Vitro Cem powder(DFL, Rio de Janeiro, Brazil)were mixed with 5 parts Vitro Cem liquid (DFL, Rio de Janeiro, Brazil) to form a cement.

### 4.2. Glass ionomer cement for luting (according to the invention)

8.1 parts Vitro Cem powder(DFL, Rio de Janeiro, Brazil) were pre-mixed with 0.9 parts of the Nano-Fluorapatit of example 1, modified as described in example 2 and than mixed with 5 parts Vitro Cem liquid (DFL, Rio de Janeiro, Brazil) to form a cement.

### 4.3. Glass ionomer cement for core build-up (comparison example)

9 parts Core Vitro Fil powder(DFL, Rio de Janeiro, Brazil) were mixed with 3 parts Core Vitro Fil liquid (DFL, Rio de Janeiro, Brazil) to form a cement.

### 4.4. Glass ionomer cement for core build-up (according to the invention)

8.1 parts Core Vitro Fil powder(DFL, Rio de Janeiro, Brazil) were premixed with 0.9 parts of the Nano-Fluorapatit of example 1, modified as described in example 2 and than mixed with 3 parts Core Vitro Fil liquid (DFL, Rio de Janeiro, Brazil) to form a cement.

The cured cements were tested and measured for strength (flexural strength) and stability in mouth (solubility in lactic acid). Results see table 7.

**Table 7**

| Example | Luting cements | | Core build-up cements | |
|---|---|---|---|---|
| | 7.1 (comparison) | 7.2 (according the invention) | 7.3 (comparison) | 7.4 (according the invention) |
| Flexural Strength [MPa] | 11,2 | 16,9 | 15,4 | 27,5 |
| Lactic acid solubility [mm] | 0,30 | 0,21 | 0,28 | 0,18 |

### Conclusions:

The introduction of nano apatite improved the flexural strength and reduced the solubility in lactic acid for these nano fluoroapatite-containing classical glass ionomer cements.

The results show that beside of bioactive aspects the flexibility and durability of the cements could be improved by additions of nano apatite.

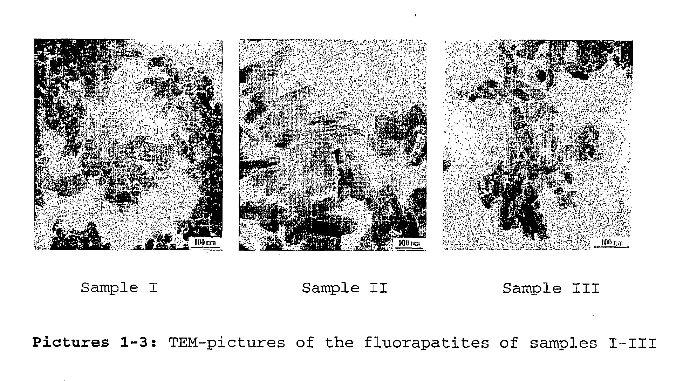

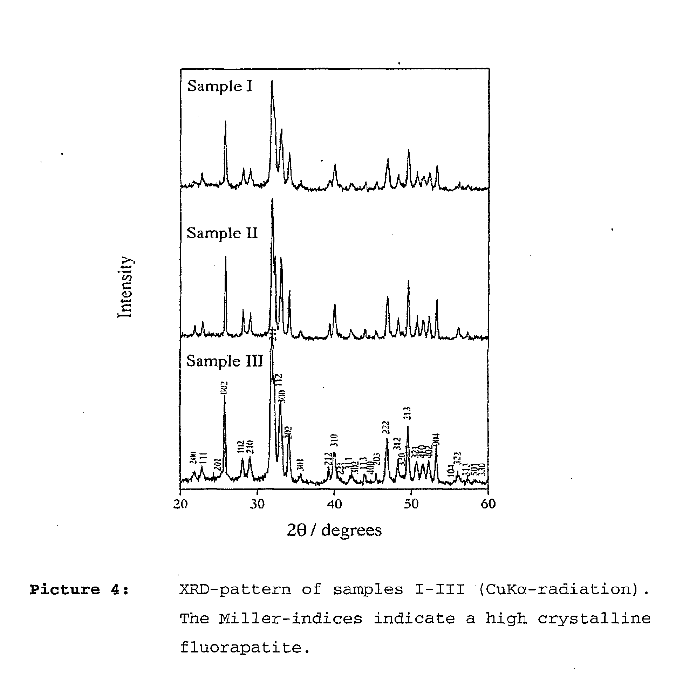

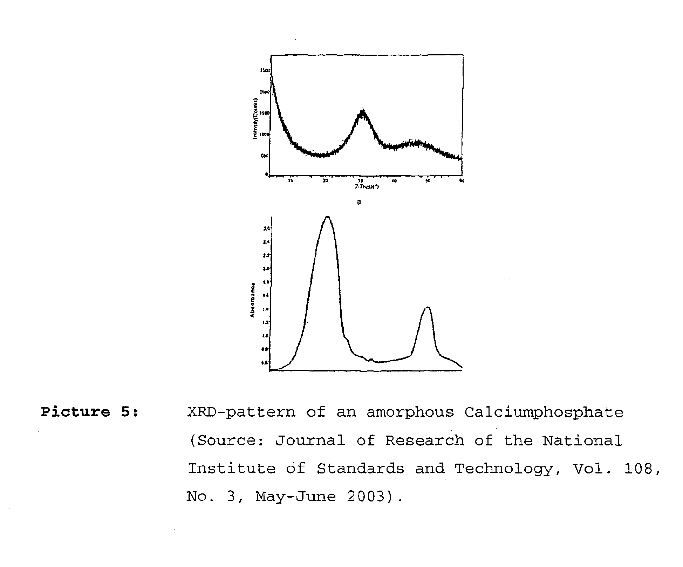

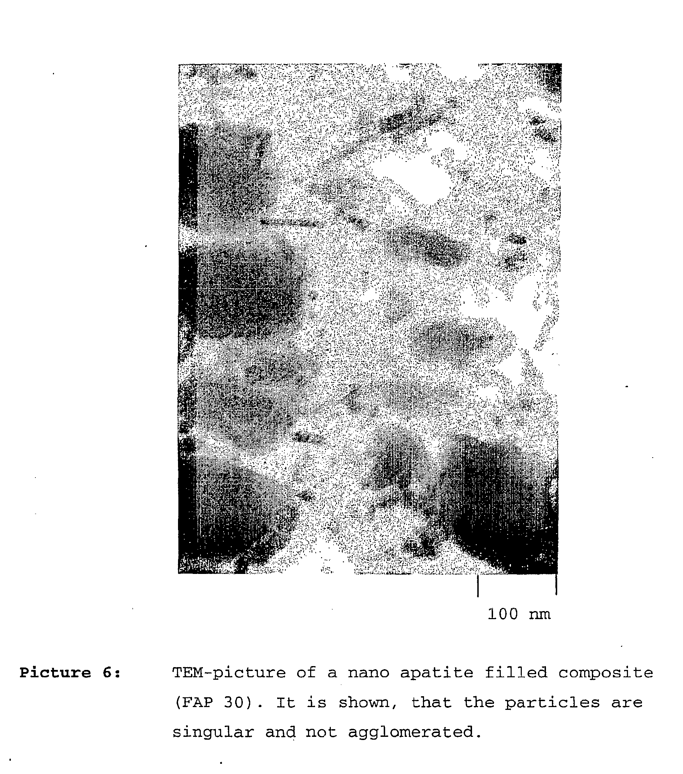

## Claims

1. Composition for restoring bone and teeth, comprising a mixture of a crosslinkable resin and/or hardenable acid/base cement system, an apatite and optionally additional fillers wherein the apatite is of the general formula:
Ca₁₀(PO₄)₆A_{z}(OH)_{2-z}
wherein A is F⁻ wherein at least 50 wt.-% of the apatite-particles have a particle size within the range from 2 to 200 nm, and wherein z=0 (Hydroxyapatite) or z=2 (Fluorapatite).

2. Composition according to claim 1 wherein the crosslinkable resin is based on free radical polymerizable monomers.

3. Composition according to claim 2 wherein the free radical polymerizable monomers are (meth)acrylates.

4. Composition according to claim 1 wherein the crosslinkable resin is based on resins polymerizable by ring-opening mechanisms.

5. Composition according to claim 4 wherein the crosslinkable resin is based on epoxy-monomers.

6. Composition according to claim 1 wherein the crosslinkable resin is based on crosslinkable silicones.

7. Composition according to claim 6 wherein the crosslinkable resin are addition curing vinyl silicones.

8. Composition according to claim 1 wherein the hardenable acid/base-cement system is that of polyalkenoate cements.

9. Composition according to claim 1 wherein the hardenable acid/base-cement system is that of silicate cements.

10. Composition according to claim 1 wherein the hardenable acid/base-cement system is that of phosphate cements.

11. Composition according to claim 1 wherein the hardenable acid/base-cement system is that based on zinc oxide and/or other metal oxides and/or weak organic acids and/or phenol-derivatives.

12. Composition according claim 1-11 wherein the apatite is surface treated.

13. Composition according to claim 12 wherein the apatite is surface treated with esters of phosphoric, phosphonic and/or carboxylic acids.

14. Composition according to any one of claims 1 - 13 which contains 1 - 70 wt.-% of the apatite.

15. Composition according to any one of claims 1 - 13 which contains 1 - 30 wt.-% of the apatite.

16. Composition according to any one of claims 1 - 13 which contains 1 - 15 wt.-% of the apatite.

17. Composition according to any one of claims 1 - 16 which contains an additional filler like powders of silica and/or glass-ceramic and/or glasses and/or microbeads and/or inorganic fibres and/or organic fibres.

18. Composition according to claim 17 wherein the refractive index of the additional filler is from 1,45 to 1,54.

19. Composition according to any one of claims 1 - 18 wherein the total load of apatite and additional filler is from 1 to 95 wt.%.

20. Method for the preparation of a composition according to any one of the preceding claims wherein a mixture of a crosslinkable resin and/or a hardenable acid/base-cement system, an apatite and optionally additional fillers is hardened and ground to a filler size of 0,2 µm to 100 µm and used as a filler.

## Patentansprüche

1. Zusammensetzung zum Restaurieren von Knochen und Zähnen, umfassend eine Mischung aus einem vernetzbaren Harz und/oder einem härtbaren Säure/Base-Zementsystem, ein Apatit und optional zusätzliche Füllstoffe, wobei der Apatit die folgende allgemeine Formel aufweist:
Ca₁₀(PO₄)₆A_{z}(OH)_{2-z}
wobei A F⁻ ist, wobei mindestens 50 Gewichts-% der Apatitteilchen eine Teilchengröße im Bereich von 2 bis 200 nm aufweisen, und wobei z = 0 (Hydroxyapatit) oder z = 2 (Fluorapatit) ist.

2. Zusammensetzung nach Anspruch 1, wobei das vernetzbare Harz auf Monomeren basiert, die durch freie Radikale polymerisierbar sind.

3. Zusammensetzung nach Anspruch 2, wobei die Monomere, die durch freie Radikale polymerisierbar sind, (Meth)Acrylate sind.

4. Zusammensetzung nach Anspruch 1, wobei das vernetzbare Harz auf Harzen basiert, die durch ringöffnende Mechanismen polimerisierbar sind.

5. Zusammensetzung nach Anspruch 4, wobei das vernetzbare Harz auf Epoxy-Monomeren basiert.

6. Zusammensetzung nach Anspruch 1, wobei das vernetzbare Harz auf vernetzbaren Siliconen basiert.

7. Zusammensetzung nach Anspruch 6, wobei das vernetzbare Harz additionshärtende Vinylsilicone sind.

8. Zusammensetzung nach Anspruch 1, wobei das härtbare Säure/Base-Zementsystem das der Polyalkenoatzemente ist.

9. Zusammensetzung nach Anspruch 1, wobei das härtbare Säure/Base-Zementsystem das der Silicatzemente ist.

10. Zusammensetzung nach Anspruch 1, wobei das härtbare Säure/Base-Zementsystem das der Phosphatzemente ist.

11. Zusammensetzung nach Anspruch 1, wobei das härtbare Säure/Base-Zementsystem das ist, das auf Zinkoxid und/oder anderen Metalloxiden und/oder schwachen organischen Säuren und/oder Phenolderivaten basiert.

12. Zusammensetzung nach Anspruch 1 bis 11, wobei der Apatit oberflächenbehandelt ist.

13. Zusammensetzung nach Anspruch 12, wobei der Apatit mit Estern von Phosphor-, Phosphon- und/oder Carbonsäuren oberflächenbehandelt ist.

14. Zusammensetzung nach einem beliebigen der Ansprüche 1 bis 13, die 1 - 70 Gew.-% des Apatits enthält.

15. Zusammensetzung nach einem beliebigen der Ansprüche 1 bis 13, die 1 - 30 Gew.-% des Apatits enthält.

16. Zusammensetzung nach einem beliebigen der Ansprüche 1 bis 13, die 1 - 15 Gew.-% des Apatits enthält.

17. Zusammensetzung nach einem beliebigen der Ansprüche 1 bis 16, die einen zusätzlichen Füllstoff wie Pulver aus Siliziumoxid und/oder Glasskeramik und/oder Gläser und/oder Mikrokugeln und/oder anorganische Fasern und/oder organische Fasern enthält.

18. Zusammensetzung nach Anspruch 17, wobei der Brechungsindex des zusätzlichen Füllstoffs von 1,45 bis 1,54 beträgt.

19. Zusammensetzung nach einem beliebigen der Ansprüche 1 bis 18, wobei der Gesamtgehalt von Apatit und zusätzlichem Füllstoff von 1 bis 95 Gew.-% beträgt.

20. Verfahren für die Herstellung einer Zusammensetzung nach einem beliebigen der vorhergehenden Ansprüche, wobei eine Mischung eines vernetzbaren Harzes und/oder eines härtbaren Säure/Base-Zementsystems, ein Apatit und optional zusaätzliche Füllstoffe gehärtet und zu einer Füllstoffgröße von 0,2 µm bis 100 µm gemahlen und als ein Füllstoff verwendet wird.

## Revendications

1. Composition permettant de reconstruire des os et des dents, comprenant un mélange d'une résine réticulable et/ou d'un système de ciment acide/base durcissable, une apatite et éventuellement des charges additionnelles où l'apatite répond à la formule générale :
Ca₁₀(PO₄)₆A_{z}(OH)_{2-z}
où A représente F⁻, où au moins 50% en poids des particules d'apatite ont une taille de particules se trouvant dans la plage allant de 2 à 200 nm, et
où z = 0 (hydroxyapatite) ou z = 2 (fluorapatite).

2. Composition selon la revendication 1, dans laquelle la résine réticulable est à base de monomères polymérisables par radicaux libres.

3. Composition selon la revendication 2, dans laquelle les monomères polymérisables par radicaux libres sont des (méth)acrylates.

4. Composition selon la revendication 1, dans laquelle la résine réticulable est à base de résines polymérisables par des mécanismes d'ouverture de cycle.

5. Composition selon la revendication 4, dans laquelle la résine réticulable est à base de monomères époxy.

6. Composition selon la revendication 1, dans laquelle la résine réticulable est à base de silicones réticulables.

7. Composition selon la revendication 6, dans laquelle la résine réticulable constitue des vinylsilicones de durcissement par addition.

8. Composition selon la revendication 1, dans laquelle le système de ciment acide/base durcissable est celui de ciments de polyalcénoate.

9. Composition selon la revendication 1, dans laquelle le système de ciment acide/base durcissable est celui de ciments à base de silicate.

10. Composition selon la revendication 1, dans laquelle le système de ciment acide/base durcissable est celui de ciments à base de phosphate.

11. Composition selon la revendication 1, dans laquelle le système de ciment acide/base durcissable est celui à base d'oxyde de zinc et/ou d'autres oxydes métalliques et/ou d'acides organiques faibles et/ou de dérivés de phénol.

12. Composition selon la revendication 1 à 11, dans laquelle l'apatite est traitée en surface.

13. Composition selon la revendication 12, dans laquelle l'apatite est traitée en surface avec des esters d'acides phosphorique, phosphonique et/ou carboxylique.

14. Composition selon l'une quelconque des revendications 1 à 13, qui contient 1 à 70% en poids de l'apatite.

15. Composition selon l'une quelconque des revendications 1 à 13, qui contient 1 à 30% en poids de l'apatite.

16. Composition selon l'une quelconque des revendications 1 à 13, qui contient 1 à 15% en poids de l'apatite.

17. Composition selon l'une quelconque des revendications 1 à 16, qui contient une charge additionnelle telle que des poudres de silice et/ou de vitrocéramique et/ou de verres et/ou de microbilles et/ou de fibres inorganiques et/ou de fibres organiques.

18. Composition selon la revendication 17, dans laquelle l'indice de réfraction de la charge additionnelle se trouve dans la plage allant de 1,45 à 1,54.

19. Composition selon l'une quelconque des revendications 1 à 18, dans laquelle la charge totale de l'apatite et de la charge additionnelle se trouve dans la plage allant de 1 à 95% en poids.

20. Procédé de préparation d'une composition selon l'une quelconque des revendications précédentes, dans lequel un mélange d'une résine réticulable et/ou d'un système de ciment acide/base durcissable, d'une apatite et éventuellement de charges additionnelles est durci et broyé jusqu'à une taille de charge allant de 0,2 µm à 100 µm et utilisé en tant que charge.
